(19) Europäisches Patentamt
European Patent Office
Office européen des brevets

(11) Publication number: **0 043 469**
**B1**

(12) **EUROPEAN PATENT SPECIFICATION**

(45) Date of publication of patent specification: **16.01.85**

(51) Int. Cl.$^4$: **G 01 N 33/72**, G 01 N 33/82, C 12 Q 1/28

(21) Application number: **81104634.1**

(22) Date of filing: **16.06.81**

(54) **Interference-resistant test device for determining a peroxidatively active substance in a test sample and method for preparing it.**

(30) Priority: **28.06.80 US 155215**

(43) Date of publication of application:
**13.01.82 Bulletin 82/02**

(45) Publication of the grant of the patent:
**16.01.85 Bulletin 85/03**

(84) Designated Contracting States:
**DE GB**

(56) References cited:
**EP-A-0 021 407**
**EP-A-0 041 188**
**BE-A- 649 682**
**FR-A-2 198 643**
**FR-A-2 390 733**
**GB-A-1 560 530**
**US-A-3 411 887**
**US-A-3 834 907**
**US-A-4 071 317**
**US-A-4 251 222**
**US-A-4 251 223**

(73) Proprietor: **MILES LABORATORIES, INC.**
**1127 Myrtle Street**
**Elkhart Indiana 46514 (US)**

(72) Inventor: **Burkhardt, Alan Elmer**
**51818 Stony Creek Drive**
**Elkhart IN 46514 (US)**
Inventor: **Tideman, Ann Marie**
**68570 Morton Drive**
**Edwardsburg MI 49112 (US)**

(74) Representative: **Senftl, Hannes, Dr. et al**
**c/o Bayer AG Konzernverwaltung RP**
**Patentabteilung**
**D-5090 Leverkusen Bayerwerk (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 043 469 B1

## Description

Background of the invention

Field of the invention

The present invention relates to a test device and its use for the determination of a peroxidatively active substance in a test sample, wherein the test device and the determination are resistant to possible adverse effects from ascorbic acid which might also be present in the sample.

Many analytical methods are presently available for detecting the presence of peroxidatively active substances in samples such as urine, fecal suspensions, and gastrointestinal contents. Hemoglobin and its derivatives are typical of such "peroxidatively active" substances because they behave in a manner similar to the enzyme peroxidase. Such substances have also been referred to as *pseudoperoxidases*. Peroxidatively active substances are enzyme-like in that they catalyze the redox reaction between peroxides and such indicator compounds as benzidine, o-tolidine, 3,3',5,5'-tetramethylbenzidine, 2,7-diaminofluroene or similar indicator substances, thereby producing a detectable response such as a color change. Hence, most methods for determining the presence of occult blood in test samples rely on this *pseudoperoxidase* activity.

Description of the prior art

Several methods have evolved over the years which rely on enzyme-like catalysis of the peroxidic oxidation of color-forming indicators. Primarily these include wet chemical procedures and "dip-and-read" type reagent-bearing strips. Of the former, a typical example is set forth in Richard M. Henry, et al., *Chemical Chemistry Principles and Techniques* (Hagerstown, Maryland: Harper and Row, 1974), pp. 1124—1125. This procedure involves the use of glacial acetic acid (buffer), diphenylamine (indicator), and hydrogen peroxide. While such wet methods have proven analytical ability, they are nevertheless fraught with obvious shortcomings, not the least of which are poor reagent stability, inadequate sensitivity, and susceptibility to interference from ascorbate.

A second method for the determination of peroxidatively active substances, and the one presently preferred by most clinical assayists and analysts, utilizes the so-called "dip-and-read" reagent strips. Typical of such devices are reagent strips manufactured by the Ames Division of Miles Laboratories, Inc. and sold under the name Hemastix®. These comprise, in essence, a porous paper matrix affixed to a plastic strip or handle. The matrix is impregnated with a buffered mixture of an organic hydroperoxide and o-tolidine. Upon immersion in a liquid containing hemoglobin, myoglobin, erythrocytes or other *pseudoperoxidases*, a blue color develops in the matrix, the intensity of which is proportional to the concentration of the peroxidatively active substance in the sample. Thus, by comparing the color developed in the matrix to a standard color chart, the assayist can determine, on a semiquantitative basis, the amount of analyte present in the sample.

The advantages of reagent strips over wet chemistry methods are predominantly twofold: strips are easier to use because neither the preparation of reagents nor the attendant apparatus is required; and greater stability of reagents is afforded, resulting in improved accuracy, sensitivity and economy.

In the case of urinalysis, however, the recent popularity of diets which include high dosages of vitamin C (ascorbic acid) has led to serious problems in analyzing for such urine constituents as occult blood, since patients on such diets invariably have atypically elevated levels of urinary ascorbate, and since ascorbate interferes with the test.

The adverse effects of reducing agents such as ascorbate were recognized as early as 1938. R. Kohn and R. M. Watrous, *Journal of Biological Chemistry*, 124, 163—168 (1938). That the same problem still plagues this area of analysis is evidenced by a proposal in 1979 that when an occult blood (*pseudoperoxidase*) analysis in urine is performed, a simultaneous ascorbate analysis be performed in order to gauge the accuracy of the occult blood determination. L. Nielsen, P. J. Jorgensen and A. C. Hansen, *Ugeskrift for Laeger*, 141, 791—793 (1979).

Although many attempts at removing ascorbate interference with other test systems, such as glucose-sensitive reagents, are reported in the literature, to date no successful attempts have been reported whereby the determination of peroxidatively active substances has been rendered immune to these adverse effects. With the glucose-sensitive systems, approaches range from filtering out ascorbate before it reaches the reagents to utilizing an enzyme to decompose it *in situ*.

Thus, Canadian Patent No. 844,564, issued to Dahlqvist on June 16, 1970, discloses a device for glucose determination in urine or other media which includes, in addition to a porous portion impregnated with normal glucose-responsive reagents, an additional portion to receive the urine test sample. The sample-receiving portion comprises an ion exchange material, whose singular function in the device is to adsorb any ascorbate which might be present in the urine sample.

Another approach to alleviating ascorbate interference is reflected in United States Patent No. 4,168,205, which issued on September 18, 1979, to Danninger et al. This reference suggests incorporating the enzyme ascorbate oxidase into the test reagent formulation, the theory being that if ascorbate is present in the sample it will be enzymatically oxidized to dehydroascorbate, a compound which does not adversely effect the desired analysis.

United States Patent No. 3,411,887, which issued to Ku on November 19, 1968, describes a way

of eliminating ascorbate interference with reagent systems which rely on enzymatic oxidizing substances such as glucose oxidase. An ascorbate "trapping system" is employed. This comprises an "ionizable heavy metal compound which, when in an ionized state possesses an oxidation-reduction potential $E^{o}_{red}$ between that of the redox indicator dye... and that of [ascorbate]".

Many metals are cited as examples, including cobalt, iron, mercury and nickel.

In addition to these studies, attention to the ascorbate problem with glucose tests is manifested by:

1. H. Gifford, et al., *J. Amer. Med. Assoc., 178,* 149—150 (1961).
2. P. O'Gorman, et al., *Brit. Med. J.,* 603—606 (1960).
3. R. Brandt, et al., *Clin. Chem. Acta, 51,* 103—104 (1974).
4. R. Brandt, et al., *Am. J. Clin. Pathol., 68,* 592—594 (1977).

Like the above-cited Ku patent other references deal with the complexing and oxidation of ascorbate using cobalt. G. Bragagnolo (*Ann. Chim. Applicata,* 31, 350—368, 1941) reported that solutions of ascorbic acid were oxidized by air in the presence of cobalt metal. Similar activity has been reported for $Co(NH_3)_6Cl_3$ by Tomokichi Iwasaki in *Journal of the Chemical Society of Japan,* 63, 820—826 (1942).

Significantly, although the prior art deals extensively with glucose analysis, it appears bereft of suggestions as to how to solve the ascorbate interference problem with the determination of peroxidatively active substances such as peroxidase and occult blood (hemoglobin). The disclosure in U.S. Patent No. 3,411,887 (see above) notwithstanding. The prior art unequivocally teaches that metal ions, such as Co(III), are in fact *pseudoperoxidases.* For example, Co(III) acetate is used commercially to catalytically decompose cumene hydroperoxide. *The Merck Index*, 9th Ed., Page 311 (1976). A series of Co(III) complexes are reported to catalytically decompose peroxides by Kh. Lohs., *Monatsber, Deut. Akad. Wiss. Berlin, 8,* 657—659 (1966) (see *Chemical Abstracts, 67,* 120383z, 1967).

As is stated *supra*, the present invention deals with improving the present state-of-the-art system for determining peroxidatively active substances. Such systems invariably comprise an organic hydroperoxide such as cumene hydroperoxide, and a redox indicator such as o-tolidine or 3,3′,5,5′-tetramethyl benzidine. The analyte, because it mimics the enzyme peroxidase, causes a reaction between the indicator and organic hydroperoxide which yields a color, the intensity of which is a barometer of the analyte concentration. In light of the unmistakable prior art teachings of peroxidase activity shown by Co(III) complexes, one skilled in the art clearly would not expect such a substance to be compatible with the peroxide/indicator system. If one incorporates an analyte into the very reagent formulation designed to change color in the presence of that analyte, it is to be expected that false positive results would be obtained. These conclusions notwithstanding, it has surprisingly been found that the peroxidatively active Co(III) complexes not only fail to give false positive results, but they actually improve the reagent system, making it even more dependable, i.e., less sensitive to the inaccuracies caused by ascorbate interference.

The earlier European patent application 81103911.4 refers to an improved composition for detecting the presence of a peroxidatively active substance in a test sample, the composition comprising an organic hydroperoxide and an indicator capable of providing a detectable response in the presence of peroxide and the peroxidatively active substance, wherein the composition is rendered resistant to adverse effects of ascorbate by the presence of a complex of Co (III). The compositions disclosed in the working Examples also comprise 6-methoxy-quinoline.

## Summary of the invention

Briefly stated, the present invention relates to a test device for detecting the presence of a peroxidatively active substance in a test sample, wherein the device is resistant to the interfering effects of ascorbate present in the sample, the device comprising a carrier matrix incorporated with a Co(III) complex, and an indicator which is capable of providing a color change or change in the amount of light absorbed or reflected from the device in the presence of a peroxidatively active substance, characterized in that the carrier matrix is incorporated with a fused ring compound having the structure

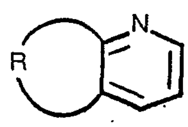

in which R is the residue of a substituted or unsubstituted carbocyclic or heterocyclic ring system, the ring having 4 to 7 atoms with the proviso that said compound is not 6-methoxy-quinoline.

The present invention also relates to a method for preparing a test device for determining the presence of a peroxidatively active substance in a test sample, wherein the device is resistant to the interfering effects of ascorbate present in the sample, characterized in that the method comprises the sequential steps of

a) preparing a first reagent mixture comprising an organic hydroperoxide and an indicator in a suitable solvent, the indicator being capable of providing a color change or change in the amount of light absorbed or reflected from the device in the presence of the hydroperoxide and the peroxidatively active substance,
b) contacting the first reagent mixture with a carrier matrix,
c) drying the carrier matrix, thereby incorporating with it the organic peroxide and the indicator,
d) preparing the second reagent mixture comprising an aqueous solution of a Co(III) complex and a fused ring compound having the structure

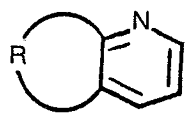

in which R is the residue of a substituted or unsubstituted carbocyclic or heterocyclic ring system, the ring having 4 to 7 atoms, with the proviso that said compound is not 6-methoxy quinoline,
e) contacting said second reagent mixture with said carrier matrix and
f) drying said carrier matrix.

Detailed description of the invention

The organic hydroperoxide contemplated for use in the test composition can be selected from many well-known organic hydroperoxides. It must, however, be capable of interacting with a peroxidatively active substance in the presence of an indicator to produce a detectable response such as a color change or change in the amount of light absorbed or reflected by the test composition. Among hydroperoxides which have been found suitable are t-butyl hydroperoxide, cumene hydroperoxide, diisopropylbenzene hydroperoxide, 2,5-dimethylhexane-2,5-dihydroperoxide, paramenthane hydroperoxide or mixtures thereof. Of these, cumene hydroperoxide has been found to be most preferable.

There exist many indicators which are capable of producing a detectable response in the presence of an organic hydroperoxide and a peroxidatively active substance and which are, therefore, suitable for use in the present invention. For the most part, these include the so-called "benzidine-type" compounds. Typical of these are benzidine, o-tolidine, 3,3',5,5'-tetra(lower alkyl)benzidine, 2,7-diaminofluorene or mixtures of these in varying proportions. By "lower alkyl" is meant an alkyl radical having 1 to 6 carbon atoms, including methyl, ethyl, *n*-propyl and isopropyl, and the various butyl, pentyl and hexyl isomers.

The Co(III) complexes useful in the present invention include $Co(NH_3)_6Cl_3$, cobalt (III) acetylacetonate, $[Co(NH_3)_5H_2O]Cl_3$, $[Co(NH_3)_5CO_3]NO_3$, $[Co(NH_3)_4CO_3]NO_3 \cdot 3H_2O$ and others. Of course, it is understood that many other cobalt (III) complexes are adaptable to the invention given the teachings herein. It has been found that $Co(NH_3)_6Cl_3$ provides excellent results, and is the preferred complex for achieving abatement of ascorbate interference. In a preferred embodiment of the present invention, the composition comprises cumene hydroperoxide, 3,3',5,5'-tetramethylbenzidine and $Co(NH_3)_6Cl_3$.

The fused ring compound of the present invention is defined broadly as having the structure

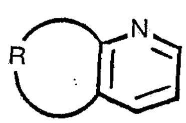

in which R is the residue of a carbocyclic or heterocyclic ring, and which may be substituted or unsubstituted with the exception of

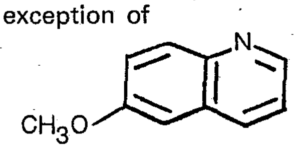 , R = 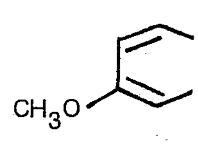

6-Methoxyquinoline

Compounds among those included in this definition, and their counterparts with respect to R in the above structure, are as follows:

I 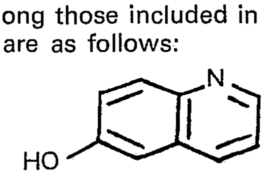 , R = 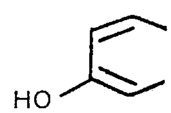

6-Hydroxyquinoline

II 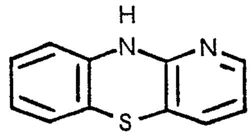 , R = 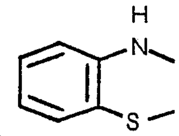

10H-Pyrido[3,2-b] [1,4] benzothiazine

III 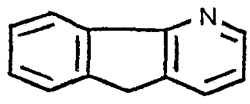 , R = 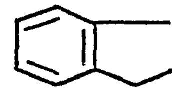

4-Azafluorene

IV 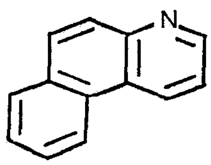 , R = 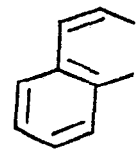

5,6-Benzoquinoline

The above compounds demonstrate that R can be carbocyclic (I, III and IV) or heterocyclic (II). Mixtures of these compounds can also be used. Moreover, the Examples, *infra*, illustrate not only the efficacy of these compounds, but also the wide diversification of substitution permissible with respect to substitution of the R residue.

Preparation of the test device includes incorporating the composition with a suitable carrier matrix, and the latter can take on a multitude of forms. Thus, U.S. Patent No. 3,846,247 teaches the use of felt, porous ceramic strips, and woven or matted glass fibers. Additionally, U.S. Patent No. 3,552,928 teaches the use of wood sticks, cloth, sponge material, and argillaceous substances. The use of synthetic resin fleeces and glass fiber felts as a carrier matrix is suggested in British Patent No. 1,369,139. Another British Patent No. 1,349,623, proposes the use of light-permeable meshwork of thin filaments as a cover for an underlying paper matrix. Polyamide fibers are taught in French Patent No. 2,170,397. The usefulness of these suggestions in the present invention notwithstanding, however, the material predominantly used in the art as a carrier matrix, and that which is especially suitable for the present invention, is a bibulous paper such as filter paper. It can thus be seen that there is a great deal of leeway in selecting an appropriate material for use as a carrier matrix, and the matrix can take on various physical forms. All of these types are intended as being within the scope of the present invention.

The ingredients of the present invention can be incorporated with the carrier matrix in a variety of ways. They can be dissolved or suspended in such suitable solvents as water, dimethylformamide, chloroform, methylene chloride, methanol, cyclohexane or mixtures thereof. Such a solution or suspension can then be used (a) to impregnate filter paper, (b) as an ink wherein the reagents are printed on a suitable matrix, or (c) the carrier matrix can be coated with the composition, such as with a doctor blade. These and other means of contacting the carrier matrix are within the purview of the invention herein.

The presently preferred method is to impregnate filter paper with a solution or suspension of the composition, the preferred solvent being distilled or deionized water used alone or mixed with dimethylformamide. Impregnation can be accomplished by dipping a piece of filter paper into the appropriate solution and drying the dipped paper in an air oven. The dried paper is then cut into a square measuring about 0.5 cm on a side, which is then mounted on one end of a polystyrene film strip measuring about 0.6×10 cm. Mounting is accomplished through use of double faced adhesive tape, such as that available from the 3M Co., known as Double Stick.

Especially preferred in formulating the device is the method wherein the organic hydroperoxide and indicator are introduced into the filter paper as a first mixture or solution. The Co(III) complex and fused ring compound are then subsequently applied as an aqueous second mixture or solution, with drying after each application. Such a two-dip process, where the carrier matrix is impregnated with the Co(III) complex in the second dip, has been found preferable, although ascorbate resistance can also result from methods such as where the Co(III) complex is applied as the first dip or all the ingredients are mixed together as a single dip.

The following examples are provided to further illustrate the concepts and advantages of the presently disclosed invention. They show how to make and use the invention, they present comparative

data demonstrating the improved ascorbate resistance it provides, and they illustrate the presently preferred embodiments thereof. These examples are, however, not to be interpreted as limiting in any way the scope of the invention.

Examples

Various fused ring compounds were employed (as were various Co(III) complexes) in preparing test devices of the present invention. These were then tested in urine samples containing varying quantities of ascorbate and occult blood to determine the effects of ascorbate on the efficacy of the devices. Finally, experiments were performed to study the order of addition of reagents to the carrier matrix and its effect on the resistance of the test device to ascorbate interference.

A. Various fused ring compounds

Example I

6-Hydroxyquinoline

This experiment illustrates the preparation of a test device using 6-hydroxyquinoline.

A first dip solution was prepared by mixing 0.12 grams of $Co(NH_3)_6Cl_3$ in 80 ml distilled water. Next, a square of Eaton and Dikeman filter paper (No. 222) measuring 4×4 in. was immersed in the first dip solution and dried in an air oven at 95°C for 10 minutes.

A second dip solution was prepared by mixing the following ingredients in the order as listed.

| | |
|---|---|
| Distilled water | 35 ml* |
| Sodium citrate | 1.50 g** |
| Citric acid | 1.92 g |
| Triethanolamine borate | 3.5 g |
| Ethylenediaminetetracetic acid (tetrasodium salt) | 0.10 g |
| Methyl sulfone | 4.66 g |
| Sodium dodecyl sulfate | 0.52 g |
| Dimethyl formamide | 35 ml*** |
| 3,3′,5,5′-Tetramethylbenzidine | 0.42 g*** |
| Cumene hydroperoxide | 1.4 g |

* milliliters
** grams
*** these ingredients combined together before addition to the mixture.

To a 10 ml aliquot of this solution was added 0.036 g of 6-hydroxyquinoline.

The filter paper square containing the first dip residue was then immersed in the 10 ml aliquot of second dip solution and dried at 95°C for 10 minutes in an air oven.

Assembly of the test device comprised applying a 0.6 centimeter (cm) square of the dried, impregnated paper to one end of a polystyrene film strip measuring 0.6 by 10 cm using double faced adhesive tape (3M Company, Double Stick 415).

Testing of the device in urine containing both hemoglobin and ascorbate yields easily discernible color levels corresponding to various hemoglobin concentrations.

Example II

5,6-Benzoquinoline

A test device was prepared by following the procedure of Example I, except that instead of using 6-hydroxyquinoline, 0.045 g of 5,6-benzoquinoline was added to a 10 ml aliquot of the second dip solution. When the resultant device is tested in urine containing both hemoglobin and ascorbate, easily discernible color levels corresponding to the hemoglobin concentrations are observed.

Example III

4-Azafluorene

A test device was prepared by following the procedure of Example I, except that, instead of using 6-hydroxyquinoline, 0.042 g of 4-azafluorene was added to a 10 ml aliquot of the second dip solution.

When the resultant device is tested in urine containing both hemoglobin and ascorbate, easily discernible color levels corresponding to the hemoglobin concentrations are observed.

Example IV
10H-Pyrido[3,2-b][1,4]benzothiazine

A test device was prepared by following the proceudre of Example I except that, instead of using 6-hydroxyquinoline, 0.050 g of 10H-pyrido[3,2-b][1,4]benzothiazine was added to a 10 ml aliquot of the second dip solution. When the resultant device is tested in urine containing both hemoglobin and ascorbate, easily discernible color levels corresponding to the hemoglobin levels are observed.

B. Ascorbate resistance testing
Example V
Effects of various fused ring compounds

A series of experiments was conducted in order to study the effects of ascorbate on the test devices of the present invention.

Test devices were prepared as described in Examples I—IV. In addition, control devices were prepared by impregnating the filter paper carrier matrix with the following dip:

| | |
|---|---|
| Distilled water | 50 ml |
| Sodium citrate | 2.13 g |
| Citric acid | 2.77 g |
| Triethanolamine borate | 5.00 g |
| Methyl sulfone | 6.67 g |
| Sodium lauryl sulfate | 0.75 g |
| Ethylenediaminetetracetic acid | 0.13 g |
| Dimethylformamide | 50.0 ml |
| 6-Methoxyquinoline | 0.4 ml |
| Cumene hydroperoxide | 2.0 ml |

These devices were assessed by dipping into test samples comprising negative urine, and aliquots thereof to which had been added human whole blood, ascorbic acid or both.

The appearance of color was noted visually after one minute and assigned a numerical value corresponding to standard relative color intensity values. A control strip was dipped into urine samples containing various concentrations of hemoglobin, but no ascorbate. Standard color values were assigned as follows

| | | | | | |
|---|---|---|---|---|---|
| Hemoglobin (mg* %) | 0 | 0.015 | 0.030 | 0.135 | 0.405 |
| Color value | 0 | 10 | 20 | 30 | 40 |

* milligrams per deciliter.

Thus, the color formed in the control device upon being dipped in a urine sample having no hemoglobin present was ascribed a color value of 0; whereas the color produced by a urine containing 0.405 milligrams hemoglobin per 100 milliliters was assigned the value of 40.

The results are as follows:

| | Urine sample | | Visual results after 1 min. | |
|---|---|---|---|---|
| Example | Hemoglobin mg. % | Ascorbic acid mg. % | Control | Test device |
| I | 0.030 | 0 | 20 | 10* |
| | 0.030 | 25 | 8 | 8 |
| II | 0.030 | 0 | 20 | 20 |
| | 0.030 | 25 | 8 | 15 |
| III | 0.030 | 0 | 20 | 20 |
| | 0.030 | 25 | 8 | 18 |
| IV | 0.030 | 0 | 20 | 20 |
| | 0.030 | 25 | 8 | 20 |

* anomalous data.

As can be seen from the above data, devices prepared using various fused ring compounds all possess similarly excellent resistance to ascorbate interference whereas the control devices exhibit ascorbate interference susceptibility.

Example VIII
Effects of various Co(III) complexes

Experiments similar to those of Example VII were conducted with the devices prepared in Example VI.

Instead of using the visual observation technique of Example VII, color formation was followed using a device known as the "Rapid Scanner". This device is a scanning reflectance spectrophotometer interfaced with a PDP-12 computer obtained from the Digital Equipment Corporation. The instrument is used for the rapid measurement of reflectance spectra in the visual range. The computer allows for the storage of spectral data and computations. Measurements of the performances of reagent strips in the Rapid Scanner have the following advantages over visual observations of the same strips:

1. The light source and conditions surrounding the sample remain fixed. In visual readings the light source can vary, not only in wavelength components, but also in relation to the location of the strip being observed.
2. The detector characteristics remain fixed with the Rapid Scanner. In visual observation, the detector (i.e., the eyes of the observer) varies from person to person, and with the same person, from day to day.
3. The Rapid Scanner allows more precise quantitation of the data than does human observation, thereby permitting comparisons between results to be made in a more objective manner than with visual observation.

The Rapid Scanner instrument was constructed by the Ames Division of Miles Laboratories, Inc., Elkhart, Indiana, U.S.A., from whom complete information with respect to structural and performance characteristics are obtainable.

Tri-stimulus values from the Rapid Scanner were used to calculate color difference values ($\Delta E$) according to the convention contained within "Supplement No. 2 to Commission Internationale de L'Eclairage (Paris, France) Publication No. 15, Colorimetry, (E.-1.3.1), 1971". The data from this instrument are therefore recorded below in terms of $\Delta E$, or color difference units.

Thus, as in Example VII, control devices with no Co(III) complex were compared with those from Example VI which contained various Co(III) complexes. The comparison was performed using urines containing various hemoglobin levels with and without ascorbate.

The color difference units ($\Delta E$) provided by the Rapid Scanner correspond to hemoglobin levels (in the absence of ascorbate) in accordance with the following:

| | | | | | |
|---|---|---|---|---|---|
| Hemoglobin (mg. %) | 0 | 0.015 | 0.030 | 0.045 | 0.135 |
| $\Delta E$ | 0 | 40 | 50 | 58 | 63 |

This data was obtained from the Rapid Scanner using the control devices, i.e., devices prepared as in Example VI except that no Co(III) complex was present.

When the devices of Example VI containing various cobalt (III) complexes were tested in urine samples containing 0.135 mg. % hemoglobin with and without ascorbate, the results were as follows:

| | Urine sample | | | |
|---|---|---|---|---|
| Co(III) Complex | Hemoglobin (mg. %) | Ascorbic acid (mg. %) | Control | Test device |
| A | 0.135 | 0 | 63 | 57 |
| | 0.135 | 50 | 18 | 35 |
| B | 0.135 | 0 | 63 | 60 |
| | 0.135 | 50 | 18 | 27 |
| C | 0.135 | 0 | 63 | 62 |
| | 0.135 | 50 | 18 | 43 |
| D | 0.135 | 0 | 63 | 54 |
| | 0.135 | 50 | 18 | 49 |

A=Co(III) acetylacetonate
B=$[Co(NH_3)_5H_2O]Cl_3$
C=$[Co(NH_3)_5CO_3]NO_3$
D=$[Co(NH_3)_4CO_3]NO_3 \cdot 3H_2O$

The table portrays data which evidences a dramatic reduction in susceptibility to ascorbate interference due to the presence of a Co(III) complex and a fused ring compound.

Example IX

Because of the prior art teachings of the peroxidative activity of cobalt (III), devices prepared using $Co(NH_3)_6Cl_3$ as in Example VI and the control device (prepared as in Example VI except without the cobalt complex) were tested for stability. This experiment showed virtually no difference in stability between the devices, despite the fact that one would except the cumene hydroperoxide in the composition to decompose rapidly in the presence of Co(III).

The cobalt-containing and control devices were stressed by being stored for two weeks in an air oven at 50°C. These stressed devices, as well as unstressed ones, were then dipped in negative urine and negative urine to which had been added various amounts of human whole blood. The appearance of color was evaluated as in Example VI, i.e., visually after one minute. The data is as follows

Control

| | Visual results after 1 minute | |
|---|---|---|
| Hemoglobin (mg. %) | Unstressed | 2 wks. 50°C |
| 0.000 | 0 | 0 |
| 0.015 | 20 | 19 |
| 0.030 | 22 | 21 |
| 0.045 | 25 | 25 |
| 0.135 | 30 | 32 |
| 0.405 | 40 | 40 |

Device with $Co(NH_3)_6Cl_3$

| Hemoglobin (mg. %) | Visual results after 1 minute Unstressed | 2 wks. 50°C |
|---|---|---|
| 0.000 | 0 | 0 |
| 0.015 | 20 | 18 |
| 0.030 | 21 | 21 |
| 0.045 | 25 | 23 |
| 0.135 | 32 | 30 |
| 0.405 | 40 | 40 |

As can be seen from the above data, no incompatibility between the peroxide and Co(III), even after storage at 50°C for two weeks, is evident. Moreover, the cobalt-containing test devices are equally as sensitive as the control devices without the presence of cobalt (III).

D. A method for preparing the device
Example X
The effects of ordered addition

An experiment was conducted to study the method for preparing the test device, and to explore the effect of varying the order of addition of reagents to the carrier matrix. When devices are prepared by adding the Co(III) complex as a first dip (Example VI), excellent ascorbate resistance is noted. However, when the order of addition is reversed, i.e., the complex is added as a second dip, ascorbate resistance was found to be diminished. Surprisingly, when the fused ring compound is combined with the Co(III) in the second dip, the ascorbate resistance returns to the level of devices prepared as in Example VI.

Three sets of test devices were prepared. Set (a) was prepared by incorporating $Co(NH_3)_6Cl_3$ into a paper matrix as a first dip, the remaining components, including the fused ring compound, being added as a second dip. The devices of set (b) were prepared in reverse order of addition of set (a), i.e., the cobalt complex being added as the second dip, the other ingredients comprising the first dip. Set (c) illustrates devices prepared where the second dip comprises both the cobalt complex and the fused ring compound.

Set (a)—Co(III) in first dip

Test devices were prepared wherein $Co(NH_3)_6Cl_3$ was added as an aqueous first dip, which was formulated by dissolving 0.13 g $Co(NH_3)_6Cl_3$ in 10 ml distilled water. This solution was used to impregnate a piece of laboratory filter paper (Eaton & Dikeman No. 237), which was then dried for 10 minutes in an air oven at 95°C.

A second dip solution was prepared by combining the following components in the order as listed.

| | |
|---|---|
| Distilled water | 25 ml |
| Sodium citrate | 1.07 g |
| Citric acid | 1.38 g |
| Triethanolamine borate | 3.33 g |
| Methyl sulfone | 3.33 g |
| Dimethylformamide | 25.0 ml |
| 6-Methoxyquinoline | 200 $\mu l$ |
| Cumene hydroperoxide | 1.0 ml |
| 3,3′,5,5′-Tetramethylbenzidine | 0.30 g |

To 20 ml of this solution were added

| | |
|---|---|
| Sodium lauryl sulfate | 0.20 g |
| Ethylenediamine tetraacetic acid | 0.01 g |

to make up the tetrasodium salt second dip solution.

The dried, cobalt-impregnated paper was immersed in the second dip and dried as before at 95°C for 10 minutes.

Devices prepared by this method were tested as indicated below to study their resistance to ascorbate interference when used to analyze hemoglobin in urine. The results are as follows:

| Urine sample | | |
|---|---|---|
| Hemoglobin (mg. %) | Ascorbic acid (mg. %) | Visual results |
| 0 | 0 | 3 |
| 0.135 | 0 | 30 |
| 0.135 | 50 | 20 |

The data indicates good ascorbate resistance, i.e., relatively low interference with the hemoglobin analysis.

Set (b)—Co(III) in second dip

A second set of test devices was prepared wherein the cobalt complex was added as the second dip. In this experiment a piece of the filter paper used for Set (a), above, was immersed in the second dip solution, dried, and then immersed into the cobalt-containing first dip and redried. Other than reversing the order of addition, the method was identical to that used for Set (a).

When tested as in Example IV with urines with and without hemoglobin and/or ascorbate the results were as follows:

| Urine sample | | |
|---|---|---|
| Hemoglobin (mg. %) | Ascorbic acid (mg. %) | Visual results |
| 0 | 0 | 1 |
| 0.135 | 0 | 28 |
| 0.135 | 50 | 5 |

The data indicates a dramatic susceptibility to interference by the presence of ascorbate in the test sample.

Set (c)—Co(III) and fused ring compound in second dip

A third set of test devices was prepared wherein the second dip contained both cobalt complex and the fused ring compound.

The first dip was prepared by combining the following ingredients in the order as listed:

| | |
|---|---|
| Distilled water | 50 ml |
| Sodium citrate | 2.13 g |
| Citric acid | 2.77 g |
| Triethanolamine borate | 6.67 g |
| Ethylenediaminetetraacetic acid, tetrasodium salt | 0.07 g |
| Methyl sulfone | 6.67 g |
| Sodium lauryl sulfate | 1.00 g |

To a 10 ml aliquot of this solution was added 10 ml dimethylformamide, 0.4 g cumene hydroperoxide and 0.12 g of 3,3′,5,5′-tetramethylbenzidine.

A piece of laboratory filter paper (Eaton & Dikeman No. 222) was immersed in the first dip solution and dried at 95°C for 10 minutes in an air oven, whereupon the dried paper was immersed into the second dip solution.

The second dip was prepared by dissolving 0.24 g $Co(NH_3)_6Cl_3$ in 80 ml of distilled water. To 10 ml of this solution was added 10 ml dimethylformamide followed by 80 $\mu$l 6-methoxyquinoline. Following immersion in the second dip, the filter paper was again dried for 10 minutes at 95°C.

When this test paper was immersed in urines with hemoglobin with or without ascorbate, the following data was obtained:

| Urine sample | | |
|---|---|---|
| Hemoglobin (mg. %) | Ascorbic acid (mg. %) | Visual results |
| 0.135 | 0 | 5 |
| 0.135 | 0 | 40* |
| 0.135 | 50 | 38* |

* Note these devices were more sensitive than those in sets (a) and (b) above, perhaps because E & D 222 paper was used instead of E & D 237.

The data shows the devices of set (c) have a remarkable degree of resistance to ascorbate interference when used to analyze for hemoglobin in urine.

**Claims**

1. A test device for determining the presence of a peroxidatively active substance in a test sample, wherein the device is resistant to the interfering effects of ascorbate present in the sample, the device comprising a carrier matrix incorporated with a Co(III) complex, and an indicator which is capable of providing a color change or change in the amount of light absorbed or reflected from the device in the presence of the peroxidatively active substance, characterized in that the carrier matrix is incorporated with a fused ring compound having the structure

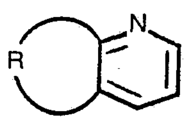

in which R is the residue of a substituted or unsubstituted carbocyclic or heterocyclic ring system, the ring having 4 to 7 atoms with the proviso that said compound is not 6-methoxy quinoline.

2. A device according to claim 1, characterized in that the Co(III) complex is $Co(NH_3)_6Cl_3$, cobalt (III) acetylacetonate, $[Co(NH_3)_5H_2O]Cl_3$, $[Co(NH_3)_5CO]NO_3$, $[Co(NH_3)_4CO_3]NO_3 \cdot 3H_2O$, or mixtures thereof, preferably $Co(NH_3)_3Cl_3$.

3. A device according to any of claims 1 or 2, characterized in that said compound is 5,6-benzoquinoline, 4-azafluorene, 10H-pyrido[3,2-b]-[1,4] benzothiazine, 6-hydroxyquinoline or mixtures thereof.

4. A method for preparing a test device for determining the presence of a peroxidatively active substance in a test sample, wherein the device is resistant to the interfering effects of ascorbate present in the sample, characterized in that the method comprises the sequential steps of

a) preparing a first reagent mixture comprising an organic hydroperoxide and an indicator in a suitable solvent, the indicator being capable of providing a color change or change in the amount of light absorbed or reflected from the device in the presence of the hydroperoxide and the peroxidatively active substance,
b) contacting the first reagent mixture with a carrier matrix,
c) drying the carrier matrix, thereby incorporating with it the organic peroxide and the indicator,
d) preparing a second reagent mixture comprising an aqueous solution of a Co(III) complex and a fused ring compound having the structure

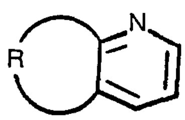

in which R is the residue of a substituted or unsubstituted carbocyclic or heterocyclic ring system, the ring having 4 to 7 atoms, with the proviso that said compound is not 6-methoxy quinoline,
e) contacting said second reagent mixture with said carrier matrix and
f) drying said carrier matrix.

5. A method according to claim 4, characterized in that the Co(III) complex is $Co(NH_3)_6Cl_3$, cobalt (III) acetylacetonate, $[Co(NH_3)_5H_2O]Cl_3$, $[Co(NH_3)_5CO]NO_3$, $[Co(NH_3)_4CO_3]NO_3 \cdot 3H_2O$, or mixtures thereof, preferably $Co(NH_3)_6Cl_3$.

6. A method according to any of claims 4 or 5, characterized in that said compound is 5,6-benzoquinoline, 4-azafluorene, 10H-pyrido[3,2-b]-[1,4]benzothiazine, 6-hydroxyquinoline or mixtures thereof.

## Patentansprüche

1. Testvorrichtung zur Bestimmung der Anwesenheit einer peroxidativ wirkenden Substanz in einer Testprobe, wobei die Vorrichtung gegenüber dem Einfluss des in der Probe vorliegenden Arcorbats resistent ist, und die Vorrichtung eine Trägermatrix, welche mit einem Co(III)-Komplex inkorporiert ist, und einen Indikator umfasst, der in der Lage ist, eine Farbänderung oder eine Änderung der von der Vorrichtung absorbierten oder reflektierten Lichtmenge in Gegenwart der peroxidativ wirkenden Substanz herbeizuführen, dadurch gekennzeichnet, dass die Trägermatrix mit einer kondensierten Ringverbindung der folgenden Struktur inkorporiert ist:

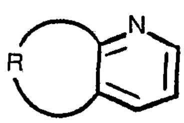

worin R den Rest eines substituierten oder nicht-substituierten carbocyclischen oder heterocyclischen Ringsystems darstellt, wobei der Ring 4 bis 7 Atome aufweist, vorausgesetzt, dass diese Verbindung nicht 6-Methoxychinolin darstellt.

2. Vorrichtung gemäss Anspruch 1, dadurch gekennzeichnet, dass der Co(III)-Komplex $Co(NH_3)_6Cl_3$, Cobalt (III)-acetylacetonat, $[Co(NH_3)_5H_2O]Cl_3$, $[Co(NH_3)_5CO]NO_3$, $[Co(NH_3)_4CO_3]NO_3 \cdot 3H_2O$, oder deren Gemische, vorzugsweise $Co(NH_3)_6Cl_3$.

3. Vorrichtung gemäss Anspruch 1 oder 2, dadurch gekennzeichnet, dass die genannte Verbindung 5,6-Benzochinolin, 4-Azafluoren, 10H-Pyrido[3,2-b]-[1,4]-benzothiazin, 6-Hydroxychinolin oder deren Gemische darstellt.

4. Verfahren zur Herstellung einer Testevorrichtung zur Bestimmung der Anwesenheit einer peroxidativ wirkenden Substanz in einer Testprobe, wobei die Vorrichtung gegenüber dem Einfluss des in der Probe vorliegenden Ascorbats resistent ist, gekennzeichnet durch die nachstehenden, aufeinanderfolgenden Schritte:

(a) Herstellung eines ersten Reagenzgemisches, welches ein organisches Hydroperoxid und einen Indikator in einem geeigneten Lösungsmittel umfasst, wobei der Indikator in der Lage ist, eine Farbänderung oder eine Änderung der von der Vorrichtung absorbierten oder reflektierten Lichtmenge in Gegenwart von Hydroperoxid und der peroxidativ wirkenden Substanz herbeizuführen,
(b) Kontaktieren des ersten Reagenzgemisches mit einer Trätermatrix,
(c) Trocknen der Trägermatrix, wobei das organische Peroxid und der Indikator in dieselbe inkorporiert werden,

(d) Herstellen eines zweiten Reagenzgemisches, welches eine wässrige Lösung eines Co(III)-Komplexes und einer kondensierten Ringverbindung der folgenden Struktur umfasst:

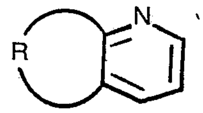

worin R der Rest eines substituierten oder nicht-substituierten carbocyclischen oder heterocyclischen Ringsystems darstellt, wobei der Ring 4 bis 7 Atome aufweist, vorausgesetzt, dass die genannte Verbindung nicht 6-Methoxychinolin darstellt,

(e) Kontaktieren des genannten zweiten Reagenzgemisches mit der genannten Trägermatrix, und

(f) Trocknen der genannten Trägermatrix.

5. Verfahren gemäss Anspruch 4, dadurch gekennzeichnet, dass der Co(III)-Komplex $Co(NH_3)_6Cl_3$, Cobalt (III)-acetylacetonat, $[Co(NH_3)_5H_2O]Cl_3$, $[Co(NH_3)_5CO]NO_3$, $[Co(NH_3)_4CO_3]NO_3 \cdot 3H_2O$ oder deren Gemische, vorzugsweise $Co(NH_3)_6Cl_3$ darstellt.

6. Verfahren gemäss Ansprüchen 4 oder 5, dadurch gekennzeichnet, dass die genannte Verbindung 5,6-Benzochinolin, 4-Azafluoren, 10H-Pyrido-[3,2-b]-[1,4]-benzothiazin, 6-Hydroxychinolin oder ein Gemisch derselben darstellt.

**Revendications**

1. Dispositif analytique pour détecter la présence d'une substance douée d'activité de peroxydase dans un échantillon à analyser, ce dispositif étant résistant aux effets d'interférence de la part de l'ascorbate présent dans l'échantillon, et comprenant une matrice de support à laquelle est incorporé un complexe de Co(III), et un indicateur qui est capable der présenter un changement de couleur ou un changement de la quantité de lumière absorbée ou réfléchie par le dispositif en présence de la substance douée d'activité de peroxydase, caractérisé en ce que la matrice de support renferme un composé à noyaux condensés répondant à la formule

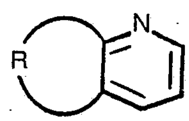

dans laquelle R est le résidu d'un noyau carbocyclique ou hétérocyclique substitué ou non substitué, le noyau ayant 4 à 7 atomes de carbone, sous réserve que ledit composé ne soit pas la 6-méthoxyquinoléine.

2. Dispositif suivant la revendication 1, caractérisé en ce que le complexe de Co(III) est $Co(NH_3)_6Cl_3$, l'acétylacétonate de cobalt (III), $[Co(NH_3)_5H_2O]Cl_3$, $[Co(NH_3)_5CO]NO_3$, $[Co(NH_3)_4CO_3]NO_3 \cdot 3H_2O$ ou leurs mélanges, de préférence $Co(NH_3)_3Cl_3$.

3. Dispositif suivant l'une des revendications 1 et 2, caractérisé en ce que ledit composé est la 5,6-benzoquinoléine, le 4-azafluorène, la 10H-pyrido[3,2-b]-[1,4]benzothiazine, la 6-hydroxyquinoléine ou leurs mélanges.

4. Procédé de préparation d'un dispositif analytique pour la détection de la présence d'une substance douée d'activité de peroxydase dans un échantillon à analyser, dans lequel le dispositif résiste aux effets d'interférence de la part d'un ascorbate présent dans l'échantillon, caractérisé en ce qu'il comporte les étapes successives de

a) préparation d'un premier mélange de réactifs comprenant un hydroperoxyde organique et un indicateur dans un solvant convenable, l'indicateur étant capable de présenter un changement de couleur ou un changement dans la quantité de lumière absorbée ou réfléchie par le dispositif en présence de l'hydroperoxyde et de la substance douée d'activité de peroxydase,

b) mise en contact du premier mélange de réactifs avec une matrice de support,

c) séchage de la matrice de support, en y incorporant ainsi le peroxyde organique et l'indicateur,

d) préparation d'un second mélange de réactifs formé d'une solution aqueuse d'un complexe de Co(III) et d'un composé à noyaux condensés répondant à la formule

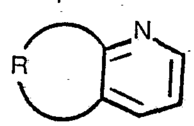

dans laquelle R est le résidu d'un noyau carbocyclique ou hétérocyclique substitué ou non substitué, le noyau ayant 4 à 7 atomes de carbone, sous réserve que ledit composé ne soit pas la 6-méthoxyquinoléine,

e) mise en contact du second mélange de réactifs avec ladite matrice de support, et

f) séchage de la matrice de support.

5. Procédé suivant la revendication 4, caractérisé en ce que le complexe de Co(III) est $Co(NH_3)_6Cl_3$, l'acétylacétonate de cobalt (III), $[Co(NH_3)_5H_2O]Cl_3$, $[Co(NH_3)_5CO]NO_3$, $[Co(NH_3)_4CO_3]NO_3 \cdot 3H_2O$ ou leurs mélanges, de préférence $Co(NH_3)_6Cl_3$.

6. Procédé suivant l'une des revendications 4 et 5, caractérisé en ce que ledit composé est la 5,6-benzoquinoléine, le 4-azafluorène, la 10H-pyrido[3,2-b]-[1,4]benzothiazine, la 6-hydroxyquinoléine ou leurs mélanges.